# FASCICULE DE BREVET EUROPEEN

(11) **EP 2 260 853 B1**
(45) Date de publication et mention de la délivrance du brevet: **21.10.2015**
(21) Numéro de dépôt: 10184844.8
(22) Date de dépôt: 28.10.2004
(51) Int. Cl.: A61K 31/737, A61K 31/795, A61K 47/36, A61K 47/42, A61P 29/00, A61P 17/02

(54) **Polymères biocompatibles pour une composition médicale**
Biokompatible Polymere für eine medizinische Zubereitung
Biocompatible polymers for a medical composition

(30) Priorité: 28.10.2003 FR 0312605; 05.04.2004 FR 0403550
(43) Date de publication de la demande: 15.12.2010
(62) Demande divisionnaire de: 10006666.1
(73) Titulaire: Organes Tissus Régénération Réparation Remplacement, 75001 Paris (FR); Barritault, Denis, 75001 Paris (FR)
(72) Inventeur: Barritault, Denis, 75001, PARIS (FR); Barbier-Chassefiere, Véronique, 91850, Bouray-sur-Juine (FR)
(74) Mandataire: Novagraaf Technologies

(56) Documents cités:
- EP-A2- 0 093 489
- FR-A- 2 781 485
- ESCARTIN Q ET AL: "A new approach to treat tissue destruction in periodontitis with chemically modified dextran polymers.", THE FASEB JOURNAL : OFFICIAL PUBLICATION OF THE FEDERATION OF AMERICAN SOCIETIES FOR EXPERIMENTAL BIOLOGY. APR 2003, vol. 17, no. 6, avril 2003 (2003-04), pages 644-651, XP008059847, ISSN: 1530-6860
- JEANBAT-MIMAUD VIVIANE ET AL: "Bioactive functionalized polymer of malic acid for bone repair and muscle regeneration", JOURNAL OF BIOMATERIALS SCIENCE POLYMER EDITION, vol. 11, no. 9, 2000, pages 979-991, XP008060107, ISSN: 0920-5063

## Description

On connaît dans l'art antérieur des composés désignés HBGFPP possédant la capacité d'accélérer les processus de réparations des lésions :
- de tissus nerveux comme décrit dans le brevet FR 94/03806,
- de tissus musculaires dans le brevet US 5,852,003, et
- du tractus digestif dans le brevet US 5,852,004.

Il a également été décrit dans le brevet FR 2781485 une famille de molécules, désignées RGTA, qui présentent des propriétés remarquables, notamment, des effets protecteurs contre les radicaux libres, des effets de prévention de la formation de fibroses et des effets de régulation de l'homéostasie tissulaire en général et des tissus osseux en particulier.

Ainsi les RGTA ont été décrits comme agents de cicatrisation de lésions de la peau, de la cornée, des os plats et longs, des tissus ischémiés comme les tissus des muscles squelettiques ou cardiaques ou nerveux, ou encore des maladies neurodégénératives ou encore des tissus irradiés. Les HBGFPP, et plus particulièrement les RGTA, sont donc des agents favorisant la cicatrisation et la régénération,

La présente invention concerne le domaine de la prévention, du soulagement ou du traitement du prurit. L'invention a plus particulièrement pour objet l'utilisation de polymères biocompatibles de formule générale AaXxYy, définie ci-après, pour la préparation d'une composition pharmaceutique, dermatologique ou cosmétique destinés à la prévention, au soulagement et/ou au traitement-du prurit et/ou à la protection des tissus contre ceux-ci. Un objet qui n'est pas inclus dans l'étendue des présentes revendications ne fait pas partie de la présente invention.

tissulaires en général quels que soient les types de lésions et ce, dans la mesure où ils agissent comme agents de potentialisation et de protection des facteurs de croissance naturellement présents dans les tissus.

Il a également été rapporté dans la demande de brevet français publiée sous le No. 2 718 025, que les HBGFPP sont des agents présentant des activités anti-inflammatoires en inhibant certaines enzymes de l'inflammation comme l'élastase leucocytaire.

Par ailleurs, la demande de brevet français publiée sous le No. 2 461 724 ainsi que le brevet US 4,740,594 décrivent les dérivés de dextran contenant des résidus carboxyméthyle, carboxyméthyle benzylamide sulfonate (dérivés carboxylés et sulfatés de dextran) présentant des activités anti-inflammatoires par inhibition du complément. Plus récemment, ces mêmes propriétés ont été rapportées pour des dérivés sulfatés de fucanes ou de dextran dépourvus de groupement benzylamine (Fisher, et al., Mol Immunol., 1994, 31 (4) P 247 et Maiga et al., Carbohydrate Polymers, 1997, 32, 89-93).

Le document Escartin et al. « A new approach to treat tissue destruction in periodontitis with chemically modified dextran polymers » The Faseb journal, vol 17, no 6, avril 2003, pages 644-651, décrit l'utilisation de RGTA pour le traitement de la pérondontite et son effet sur la destruction des tissus liés.

Indépendamment des activités de régénération, il a maintenant été mis en évidence une activité des polymères biocompatibles sur la douleur et le prurit (ou démangeaison). En effet, ces composés montrent une capacité insoupçonnée de diminuer, soulager ou supprimer la douleur et le prurit. Ces effets ont été observés dans les quelques minutes suivant l'application de polymères biocompatibles et leur action de soulagement de la douleur et le cas échéant de démangeaisons, puis l'effet du sentiment de confort qui en résulte ont duré plusieurs heures, voire plusieurs jours. Ces effets sont obtenus à chaque nouvelle application de polymères biocompatibles. Ces composés agissent non seulement sur des tissus lésés ou irrités mais également sur des tissus sains sans que les lésions tissulaires soient nécessairement visibles.

Ces effets de soulagement sur le traitement de la douleur et/ou des démangeaisons et le sentiment de confort ont été observés dans tous les types de douleur et de démangeaisons, quelles que soient l'origine de la douleur ou la nature des tissus concernés, par application locale ou systémique ou par administration par voie orale ou par voie aérienne. Ces effets ont été observés plus particulièrement sur des tissus de surface en contact direct avec l'extérieur comme la peau, la cornée, ou le tympan ou sur des tissus en contact indirect comme les muqueuses du tractus digestif, des voies respiratoires nasales et pulmonaires. Des douleurs sur des tissus plus profonds et donc plus difficilement accessibles comme les muscles, les tendons ou les articulations (genoux, coudes), ou les os du pied après chirurgie orthopédique, ont été soulagées par application locale sur la peau au niveau de la zone de souffrance, par injection locale ou systémique ainsi que par administration orale. Curieusement, l'administration orale ponctuelle a permis également de soulager des céphalées et l'administration orale quotidienne sur plusieurs semaines de traiter des souffrances chroniques du système locomoteur ou dorsales, des tissus nerveux, des muqueuses du tractus digestives ou pulmonaires, et des tissus cutanés. L'application de polymères biocompatibles peut se faire d'une manière préventive, juste après la lésion, sur le site de la lésion et prévenir ou atténuer significativement la douleur. De même, l'application sur une peau, non lésée et apparemment saine mais sensible et donnant un sentiment d'inconfort décrit dans l'expression «nerfs à fleur de peau» provoque dans certains cas un sentiment de confort et de bien être; il en est de même chez des personnes âgées ayant des peaux rugueuses et sèches dites «en peau de lézard». L'absorption par voie orale donne également cette sensation de confort et de bien-être. L'administration par voie aérienne permet également de calmer une irritation et de soulager des effets de suffocation, d'oppression respiratoire et de la toux.

Il en est de même des effets des polymères de l'invention pour calmer ou soulager les prurits, qu'ils soient locaux ou généraux. L'application locale sur des piqûres d'insecte, sur des peaux ayant de l'eczéma, du psoriasis ou tout simplement une dermatose non visible ou encore des muqueuses anales ou génitales ou encore le cuir chevelu (pour ne citer que les exemples les plus marquants), calme les démangeaisons locales. Il en de même pour des démangeaisons générales qui sont calmées après prises par voie orale.

Ces effets sont donc très différents des effets de stimulation de la cicatrisation et de régénération tissulaires décrits dans l'art antérieur tant par leur nature que par la rapidité avec laquelle ils sont perçus.

En conséquence, l'invention a pour objet une composition pharmaceutique, dermatologique ou cosmétique pour son application comme médicament destiné à la prévention, le soulagement et/ou le traitement du prurit et/ou à la protection des tissus contre ceux-ci, ladite composition comprenant un polymère biocompatible de formule générale (I) suivante

AaXxYy (I)

dans laquelle:
A représente un monomère, choisis parmi le glucose,
X représente un groupement RCOOR',
Y représente un groupement 0 ou N-sulfonate fixé sur A et répondant à l'une des formules suivante -ROSO3R', RNSO3R' dans lesquelles:
   R représente une chaîne hydrocarbonée aliphatique, éventuellement ramifiée et/ou insaturée et qui peut contenir un ou plusieurs cycles aromatiques, le radical R étant choisi parmi un groupement alkyle, allyle, aryle, linéaires ou ramifiés et R' représente un atome d'hydrogène ou un cation,
   a représente le nombre de monomères, tel que la masse desdits polymères de formule (I) est supérieure à 2000 daltons,
   x représente le taux de substitution des monomères A par des groupements X, x étant compris entre 20 et 150%,
   y représente le taux de substitution des monomères A par des groupements Y, y étant compris entre 30 et 150%.

Les monomères A, identiques ou différents, sont choisis parmi le glucose.

Les polysaccharides peuvent être d'origine végétale à simple chaîne comme la cellulose (glucose), ou les pectines (acide galacturonique), les fucanes, l'amidon ou plus complexe comme les alginates (acide galuronique et mannuronique), d'origine fungique comme le stéroglucane ou d'origine animale comme les chitines ou le chitosan (glucosamine).

La présente invention concerne, tout particulièrement, des polymères peu dégradés par les glycanases de mammifères et/ou de bactéries et présentant une faible activité anticoagulante. Par «peu dégradés par les glycanases», on entend, dans le cadre de la présente invention, des polymères qui, mis en solution avec ces glycanases, sont dégradés à moins de 50% alors que, dans les mêmes conditions, les substrats glycosaminoglycannes de mammifères sont dégradés de 100%. Une méthode de mesure est donnée dans l'exemple 1. Par «faible activité anticoagulante», on entend, dans le cadre de la présente invention, une activité anticoagulante inférieure au dixième de celle de l'héparine (<20 UI). Par conséquent, sont exclus de la présente invention les glycosaminoglycannes d'origine animale comme l'héparine et les héparanes sulfates, les chondroitines, dermatanes ou kératane sulfates et l'acide hyaluronique.

Le nombre de monomères A défini dans la formule (I) par «a» est tel que la masse desdits polymères de formule (I) est supérieure à environ 2000 daltons (ce qui correspond à 10 monomères de glucose). De plus, la masse desdits polymères de formule (I) est inférieure à environ 2000000 daltons (ce qui correspond à 10000 monomères de glucose). De façon avantageuse, la masse desdits polymères de formule (I) est comprise entre environ 30 et environ 100 kdaltons.

De façon préférée, le taux de substitution de l'ensemble des monomères A par les groupements X défini dans la formule générale (I) par «x» est compris entre environ 20 et 150%, et de préférence est de l'ordre de 50%.

De façon avantageuse, le taux de substitution de l'ensemble des monomères A par les groupements Y défini dans la formule générale (I) par «y» est compris entre environ 30 et 150%, et de préférence est de l'ordre de 100%.

Dans la définition des taux de substitutions ci-dessus, on entend par un taux de substitution x de 100%, le fait que chaque monomère A du polymère de l'invention contient statistiquement un groupement X. De même, on entend par un taux de substitution y de 100%, le fait que chaque monomère du polymère de l'invention contient statistiquement un groupement Y. Les taux de substitution supérieurs à 100% traduisent le fait que chaque monomère porte statistiquement plus d'un groupement du type considéré; à l'inverse, les taux de substitution inférieurs à 100% traduisent le fait que chaque monomère porte statistiquement moins d'un groupement du type considéré.

Le radical R dans les polymères décrits ci- dessus n'est pas la benzylamine ou la benzylamine sulfonate. En effet, la présence de benzylamine, bien que ne nuisant pas à l'action anti-douleur, n'est pas souhaitable, car elle est susceptible d'induire une toxicité des polymères biocompatible de formule (I).

Les radicaux R sont choisis parmi un groupement alkyle, allyle, aryle, linéaires ou ramifiés.

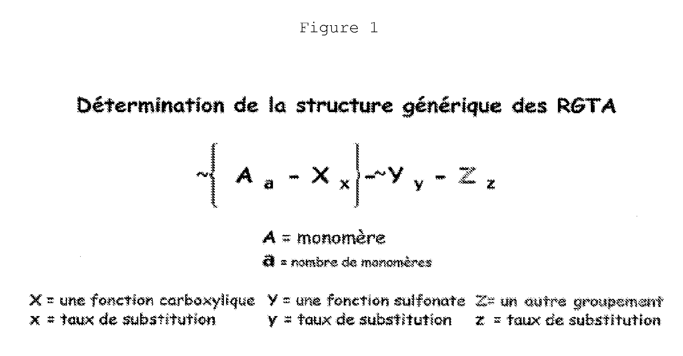

Les polymères biocompatibles utilisables dans le cadre de l'invention peuvent comprendre en outre des groupements chimiques fonctionnels Z, différents de X et Y, capables de conférer auxdits polymères des propriétés biologiques ou physicochimiques supplémentaires. La formule générale des polymères biocompatibles contenant en plus un groupe Z utilisables dans le cadre de la présente invention est donnée dans la figure 1 en annexe.

De façon préférée, le taux de substitution de l'ensemble des monomères A par des groupements Z représente par «z» dans la figure 1 est compris entre 0 et 50%, et de préférence de l'ordre de 30%.

De façon avantageuse, le groupement Z dans les polymères biocompatibles utilisables dans le cadre de la présente invention est une substance capable de conférer auxdits polymères une meilleure solubilité ou lipophilie ou encore de diminuer l'activité anticoagulante.

Dans une première forme de mise en ouvre, les groupements Z dans les polymères biocompatibles utilisables dans le cadre de la présente invention sont identiques ou différents et sont des acides aminés, des acides gras, des alcools gras, des céramides, ou des dérivés de ceux-ci, ou encore des séquences nucléotidiques d'adressage.

Dans une seconde forme de mise en oeuvre, les groupements Z dans les polymères biocompatibles utilisables dans le cadre de la présente invention sont identiques ou différents et sont des agents thérapeutiques.

Les groupements X, Y et Z peuvent être fixés directement sur le monomère A ou bien fixés les uns aux autres, l'un seulement étant fixé au monomère A.

Ainsi, les groupements Z peuvent être fixés par covalence directement sur les monomères A ou fixés par covalence sur les groupements X et/ou Y.

Mais, les groupements Z peuvent aussi être conjugués aux polymères de formule (I) par des liaisons autres que covalentes, comme des interactions ioniques ou hydrophiles, selon la nature de A, X et Y.

Lors de ses travaux, la Demanderesse a testé des polymères biocompatibles à base de glucose comme ceux dérivés du dextran, de la cellulose ou de bêta glycan ou encore à base de glucuronane ou de glucoglucuronane ou de fucanes ou encore de d'alginates. Ces polysaccharides ont été transformés en RGTA par addition de groupements carboxyliques, sulfates et des substitutions Z de différentes natures et les structures sont résumées dans le tableau 1 ci-après.

Ont également été testés des polymères biocompatibles polyesters comme le copolymère d'acide malique décrits dans la publication de Jeanbat-Mimaud et al. (« Bioactive functionalized polymers of malic acid for bone repair and muscle regeneration », Journal of Biomaterials Sciences, Polymer Edition, 2001, vol. 11, p979-991).

Ainsi, les travaux de la Demanderesse ont permis de montrer que l'application locale de polymères biocompatibles lors du traitement de lésions cutanées provenant, par exemple, de brûlures superficielles comme les expositions au soleil ou au rayonnement laser (resurfacing) ou profondes ou encore d'ulcérations liées à des pathologies vasculaires ou diabétiques induisant de fortes souffrances, ont permis de soulager les patients de ces souffrances, et ceci dans les premières heures suivant l'agression du tissu et donc bien avant que le processus de cicatrisation ne puisse être observé. Dans les cas extrêmes où ces patients étaient depuis plusieurs mois sous antalgiques puissants comme la morphine, l'effet de soulagement a été sensible rapidement après application des polymères biocompatibles et l'effet antidouleur tellement puissant qu'après une quinzaine de jours ces patients n'avaient plus besoin de traitement antalgique alors que, dans certains des exemples, ces patients étaient sous morphine depuis plus deux ans. D'une manière encore inattendue, les mêmes types de douleurs cutanées ont été soulagées après administration orale des polymères biocompatibles de l'invention.

Toujours sur les lésions cutanées, la Demanderesse a observé une diminution des souffrances liées à des gerçures ou encore aux boutons de fièvre dont les ulcérations limbiales induites étaient à la fois moins douloureuses et réduites.

Un effet marquant de soulagement de la douleur et du prurit a été observé sur des cicatrices douloureuses. Un effet associé à ce soulagement après quelques semaines d'applications itératives du polymère biocompatible est, en plus d'une douleur fortement atténuée, une amélioration esthétique de la cicatrice avec une trace plus fine, moins visible et d'une rougeur qui a diminuée ou disparue.

De même, les effets douloureux induits par des piqûres d'insectes ont été atténués. D'une manière générale, la Demanderesse a observé les effets antidouleur par simple application de polymères biocompatibles sur toutes les lésions et irritations cutanées, incluant le cuir chevelu. Les lésions pouvant être de toutes origines ainsi dans des pathologies graves comme le psoriasis ou autres maladies prenant des formes hyperkératosiques, dans les dermites de contact ou lors d'irritations mécaniques ou provoquées par des produits chimiques. Cet effet calmant du polymère biocompatible de l'invention sur les prurits et la douleur a été observé dans de nombreux cas après absorption orale ou application locale, que les prurits et/ou douleurs soient généralisés, parfois diffus comme ceux provoqués par de l'eczéma, des mycoses, des parasites, des virus comme dans le zona, ou la varicelle des médicaments, des maladies hormonales (comme le diabète, l'hyperthyroidie), ou des insuffisances rénales chroniques ou des affections hématologiques comme la maladie de Hodgkin ou polyglobulie, ou encore que les prurits ou douleurs soient locaux quelle qu'en soit l'origine.

Les mêmes observations ont été obtenues pour des souffrances d'autres tissus que la peau et notamment ceux en contact direct avec l'environnement. Ainsi lors des traitements des ulcères de cornées, nous avons observé un soulagement des animaux et une bien meilleure acceptation du traitement comme si ce traitement soulageait les douleurs liées à l'ulcération et ce dès les premières minutes suivant l'ulcération. Les effets de soulagement observé selon les cas étaient de plusieurs heures et reproduits à chacune des instillations des polymères biocompatibles.

La Demanderesse a constaté que les douleurs acides de l'estomac sont très rapidement soulagées par absorption de polymères biocompatibles. Il en est de même pour les cas de lésions des gencives, et des tissus buccaux qui, dans leur ensemble, sont rapidement soulagés après un bain de bouche ou un brossage des dents avec une solution de polymères biocompatibles. Ces effets ont été observés également en cas de rage de dent. Les aphtes ne donnent plus cette sensation de brûlure et semblent moins sensibles aux agressions comme l'exposition à des aliments acides ou caustiques. De même les irritations et prurits des muqueuses anales ou génitales sont soulagés par l'application de polymères biocompatibles en solution ou en crème.

Ce même soulagement a été observé après intoxications et brûlures pulmonaires dont les origines étaient l'inhalation de fumées provenant de feux ou des gaz toxiques. Dans ce cas, le polymère biocompatible a été inhalé après avoir été mis en aérosol. Dans le cas de personnes souffrant d'asthme, de bronchites chroniques ou aigues et d'obstruction des voies respiratoires, l'inhalation en aérosol de polymères biocompatibles de l'invention a induit un effet calmant permettant de soulager la douleur et de diminuer l'effet d'oppression respiratoire. Il a également eu pour effet de diminuer les quintes de toux et de soulager la douleur dans la gorge. Les effets sont particulièrement nets pour les personnes sensibles à la fumée de cigarettes, qui peut provoquer une irritation de la gorge et des quintes de toux, chez le fumeur comme chez celui qui le côtoie. Un effet et un soulagement ont été également observés après inhalation de polymères biocompatibles de l'invention dans le cas de personnes souffrant de rhinite, dont les muqueuses nasales sont rapidement irritées et deviennent source d'inconfort de prurit et parfois de souffrance. Ces effets antidouleur, antiprurit ou de confort ont été observés rapidement après respiration en aérosol ou en pulvérisation nasales et dès que la douleur revenant, une nouvelle exposition aux polymères biocompatibles était réalisée. D'une manière intéressante, ces inhalations étaient de moins en moins nécessaires, la douleur revenant de manière plus espacée dans le temps. De manière très surprenante, au fur et à mesure que l'effet de soulagement de la douleur ou de la gène s'observait avec le traitement par les polymères de l'invention, les maladies comme l'asthme, la rhinite allergique ou non et même la bronchite chronique se guérissaient car après plusieurs mois et arrêt de l'administration du polymère, aucun des symptômes de ces maladies ne sont revenus.

De même, la Demanderesse a observé que l'injection locale ou l'application en pommade des polymères biocompatibles au niveau des zones douloureuses des tendons comme pour le coude, la main ou encore le genou soulage rapidement et d'une manière durable la douleur. Ainsi sur plusieurs tendinites du coude comme les "tennis elbow", ou des tendinites de la main douloureuses empêchant la saisie d'objet ou les tendons ischémiés du genou comme dans la maladie de Osgood-Schlatter ou encore sur des souffrances des tendons des pieds comme le tendon d'Achille chez les sportifs des effets de soulagement des douleurs ont été observées. Le même traitement a été appliqué aux chevaux de courses souffrant de tendinites des zones articulaires des pattes. Ainsi une série d'injections dans la zone péritendineuse de quelques microlitres de polymères biocompatibles à 100 microgrammes par millilitre a permis de soulager la douleur et ce soulagement s'est manifesté par une absence de claudication après une quinzaine de jours. Le traitement a été poursuivi avec une fréquence hebdomadaire et une reprise de l'entraînement après deux mois a montré une absence de douleur à la palpation. Dans plusieurs de ces exemples, la zone douloureuse présentait au toucher une hypertrophie. Un effet intéressant du traitement a été la réduction et la suppression de cette zone au fur et à mesure de la diminution de la douleur. De même dans le cas de la maladie de Dupuytren, un soulagement de la douleur des articulations des phalanges et des métacarpiens a été observé mais et à notre surprise, l'application sur le long terme a amélioré également le fonctionnement de l'articulation et permis une récupération notable du mouvement des doigts avec une réduction visible de l'épaississement et de la rétraction de l'aponévrose palmaire. Ainsi les polymères de l'invention ont non seulement des effets sur la douleur mais améliorent l'état fonctionnel du système locomoteur, notamment dans les maladies chroniques destructrices du tissu matriciel. Comme celles observées dans les différentes formes d'arthrites, de tendinites et de la colonne vertébrale.

Des observations de soulagement ont également été observées après administration locale de polymères biocompatibles au niveau des lésions du cartilage soit par injection intra-articulaire de polymères biocompatibles seuls ou en association avec de l'acide hyaluronique ou après administration percutanée par massages autour de l'articulation. Ces mêmes effets de soulagement et parfois de suppression de douleur, ont été obtenus par absorptions itératives par voie orale de la solution de polymères biocompatibles de l'invention chez des personnes souffrant depuis longtemps de douleurs articulaires aux genoux, à la hanche et/ou encore au dos. Ce même effet de soulagement des douleurs a été observé chez des personnes souffrant de douleurs articulaires et de spasmes des membres ou des viscères ainsi que de spasmes dans des maladies neurodégénératives ou pathologies neuromusculaires aussi variées que la sclérose en plaque ou le Parkinson. De manière surprenante l'administration orale et itérative quotidienne du polymère de l'invention sur plusieurs semaines a non seulement réduit les douleurs mais la récupération partielle de la motricité a été observée dans le cas de sclérose en plaque sur une jambe quasi paralysée ainsi que la récupération presque normale d'un transit digestif. Cette récupération fonctionnelle de la motricité montre que les polymères de l'invention outre leur propriété antidouleur peuvent améliorer le traitement de maladies chroniques destructrices du tissu matriciel comme celles des gaines nerveuses.

L'effet des polymères de l'invention sur la douleur du tube digestif a également été observé dans le cas des maladies de Crohn ou des rectocolites chronique. Plusieurs patients atteints de ces maladies, sujets à des douleurs diffuses du ventre ont absorbé oralement des polymères biocompatibles, en solution dans l'eau, objets de l'invention avec des effets très nets sur la douleur ressentie au ventre. Au terme d'un à deux mois de prise biquotidienne de 50 millilitres d'une solution allant de 1 microgramme à 1 milligramme par ml de solution, ces personnes ont constaté une amélioration de leur souffrance accompagnée d'un meilleur transit et des selles normales. Ainsi, de manière surprenante le traitement de la douleur par les polymères de l'invention a amélioré également l'état de santé des malades sur une durée de temps de plusieurs mois sans signes de récidive. Ainsi le traitement des maladies chroniques du tractus digestif causant une destruction entretenue des matrices tissulaires digestives par les polymères de l'invention soulage la douleur et améliore notablement l'état de ces malades.

Des effets antalgiques ont été également observés après chirurgies orthopédiques des articulations. Ainsi la chirurgie visant à redresser les os des métatarses chez des malades atteints d'Hallux Valgus et de déformation des autres orteils sont très douloureux et le patient souffre au réveil et pendant plusieurs jours. L'injection de polymères biocompatibles par voie intramusculaire le lendemain de l'opération et après une nuit de fortes douleurs malgré une administration d'antalgiques puissants a induit un net soulagement qui s'est maintenu pendant 1 jour. L'injection réitérée le 4^{iéme} et 8^{ième} jour a permis au patient une convalescence sans douleur alors que, sans ce traitement à base de polymères biocompatibles, la douleur persiste plusieurs semaines et le soulagement du malade n'est obtenu que par un traitement continu d'antalgiques. Il en serait de même pour d'autres chirurgies.

Par conséquent, l'utilisation d'un polymère biocompatible tel que décrit ci-dessus pour la préparation d'une composition pharmaceutique ou dermatologique ou d'un dispositif médical destinés à prévenir, à soulager et/ou à traiter des douleurs induites par des lésions ou irritations ou prurits chez un individu au niveau d'une zone de contact avec un milieu extérieur est décrite.

Dans le cadre de la présente invention, on entend par «lésions ou irritations d'une zone de contact avec un milieu extérieur» aussi bien les lésions ou irritations cutanées, les lésions ou irritations ou picotement de la cornée, les lésions du tympan, les lésions ou irritations et/ou prurits du tractus digestif (lésions buccales, lésions et prurits anales, lésions stomacales, etc...), les lésions ou irritations du tractus respiratoire telles que les lésions des tissus des voies aériennes et pulmonaires et les lésions ou prurits du tractus uro-génital. De façon préférée, les douleurs des lésions, ouvertes ou cicatrisées, les irritations et/ou les prurits cutanés que visent à prévenir, à soulager et/ou à traiter les compositions ou dispositifs médicaux à base de polymères biocompatibles tels que décrits dans l'invention sont choisies parmi les lésions induites par les brûlures superficielles dues aux expositions solaires ou au rayonnement laser, les irritations et prurit du nez ou de la gorge provoquant des quintes de toux, les ulcérations liées à des pathologies vasculaires ou diabétiques, les gerçures, les ulcérations limbiales causées par des boutons de fièvre, les lésions ou prurits causées par les piqûres d'insectes, par des irritations mécaniques ou par des produits chimiques comme les acides, les maladies hyperkératosiques telles que le psoriasis ou les eczémas et dermatites de contact.

L'utilisation d'un polymère biocompatible tel que décrit ci-dessus pour la préparation d'une composition pharmaceutique ou d'un dispositif médical destinés à prévenir, à soulager et/ou à traiter des douleurs au niveau des tendons, des cartilages, des articulations et/ou du dos et d'une manière générale les douleurs associées au système locomoteur est également décrite.

Par «douleur au niveau des tendons», on entend, dans le cadre de la présente invention, aussi bien les douleurs induites par des tendinites au niveau des tendons du pied, de la main, du coude ou des articulations que les douleurs induites au niveau de tendons ischémiés comme dans la maladie de Osgood-Schlatter ou encore les douleurs induites après rupture des ligaments (tendon d'Achille, ligaments croisés du genou etc...)

Par «douleur au niveau des cartilages ou des articulations», on entend, dans le cadre de la présente invention, aussi bien les douleurs induites par des cartilages lésés au niveau des articulations comme les genoux, la hanche qu'au niveau du dos (vertèbres lombaires, cervicales et disques intervertébraux) dont les effets de douleurs transmises par les nerfs sont perçus aussi à distance dans le cou, le bras ou la jambe.

Par douleur du système locomoteur, on entend dans le cadre de la présente invention les douleurs identifiées ci-dessus des tendons et articulations mais aussi les douleurs en général diffuses ou localisées comme celles des maladies neuromotrices comme la sclérose en plaque, le Parkinson, la maladie de Lou Gehrig, les chorées, les ataxies motrices en général ou celles résultant de compressions nerveuses ou encore les neuropathies diabétiques.

L'utilisation d'un polymère biocompatible tel que décrit ci-dessus pour la préparation d'une composition pharmaceutique ou d'un dispositif médical destinés à prévenir, à soulager et/ou à traiter des douleurs au niveau des muscles et, d'une manière générale, après un choc et/ou des douleurs diffuses telles que des douleurs diffuses au niveau du ventre, de la tête comme des céphalées est également décrite.

Ainsi après un coup reçu dans la pratique d'un sport comme le rugby ou le football (aux jambes par exemple), l'application locale du polymère biocompatible a permis de supprimer ou réduire la douleur très rapidement et au sportif de reprendre son activité.

Plus généralement, les douleurs diffuses dans de nombreux cancers peuvent bénéficier du traitement par voie orale par les polymères biocompatibles en vue de soulager la douleur, sans toutefois prétendre à une action thérapeutique. Des effets de soulagement ont été observés comme dans le cas des cancers du pancréas, du foie, des reins ou dans les métastases osseuses ou pulmonaires. Le traitement par les polymères biocompatibles permettait de diminuer les doses de morphines nécessaires pour calmer les douleurs de ces malades.

La présente invention vise donc à offrir une nouvelle méthode de prévention, de soulagement et/ou de traitement du prurit et/ou de protection des tissus contre celles-ci. Ces méthodes consistent à administrer une quantité pharmaceutiquement efficace d'une composition pharmaceutique contenant un polymère biocompatible telle que précédemment décrite ou à utiliser, de façon adaptée, un dispositif médical à base d'un polymère biocompatible tel que précédemment décrit.

D'une manière surprenante l'effet du traitement de la douleur par le polymère biocompatible a conduit également dans certain cas à l'amélioration notable voire une guérison apparemment complète de la maladie ou de la lésion, elle même cause de la douleur. Ceci a été observé dans de nombreux cas jamais décrits.

Ainsi le traitement de la douleur et du prurit des cicatrices fermées récentes mais aussi anciennes a permis en outre une amélioration notable de la qualité de la cicatrice avec réduction de l'hypertrophie, de la largeur et de la rougeur de la cicatrice.

Ainsi le traitement des gènes et douleurs respiratoires a eu comme conséquences une amélioration de la maladie elle-même cause des douleurs. Cette amélioration a été notable dans le cas de traitement de mucoviscidose et dans certains cas comme dans l'asthme, la rhinite et l'emphysème pulmonaire. Une guérison apparente a été observée au moins pendant quelques mois.

Ainsi dans le cas du traitement des douleurs du système locomoteur, le soulagement de la douleur observé au fur et à mesure du traitement s'est traduit par une amélioration notable de la motricité comme dans le cas de sclérose en plaque, de polyarthrite rhumatoïde, d'arthrose, de douleur dorsales et parfois une récupération fonctionnelle totale comme dans beaucoup de tendinites. Plus surprenante a été le soulagement de la douleur dans le cas de sclérose en plaques et chez ces malades atteints de paralysie d'un membre une récupération motrice légère mais significative après quelques semaines de prise orale d'une solution aqueuse à 100 microgrammes par ml du polymère de l'invention.

Les polymères de la présente invention n'ont pas pour vocation de traiter la cause des différentes lésions provoquant la douleur ou la démangeaison, mais agissent d'une manière générale sur le tissu douloureux dans des temps courts après administration. Cette rapidité d'action ne correspond pas «a priori» à une action sur les causes elles-mêmes. Nous n'observons pas la disparition de la maladie comme le diabète même en soulageant la neuropathie associée.

D'une manière générale les maladies de toutes origines induisant sur les sites tissulaires atteints, des destructions locales de la matrice extracellulaires, que ces maladies soient chroniques ou non, d'origines infectieuses, virales ou bactériennes, d'origine auto-immune, métaboliques, ischémiques ou dégénératives ont toutes en commun l'effet d'induire une réaction locale au travers d'activations enzymatiques qui détruisent notamment les glycosaminoglycannes de la matrice.

Le polymère biocompatible est associé dans les médicaments (i.e. composition pharmaceutique ou dispositif médical) selon l'invention avec tout véhicule pharmaceutiquement acceptable connu de l'homme du métier adapté au mode d'administration utilisé. Ainsi, les médicaments selon l'invention peuvent être administrés par voie systémique, locale, orale ou en implant sous forme d'une pommade, d'une crème, d'un bain de bouche, d'un aérosol, d'une injection, etc. Les exemples qui suivent décrivent plus précisément des modes d'administration préférés des compositions utilisées dans le cadre de la présente invention.

L'utilisation d'un polymère biocompatible tel que défini précédemment pour la préparation d'une composition de confort et notamment cosmétique pour la prévention et le soulagement des gênes et désagréments cutanés tels que picotements, irritations, démangeaisons et tiraillements cutanés ainsi que pour la protection des tissus comme la peau, la cornée et les muqueuses est également décrite.

D'autres avantages et caractéristiques de l'invention apparaîtront des exemples qui suivent et qui concernent la préparation et la formulation des polymères biocompatibles utilisés dans le cadre de la présente invention et qui décrivent également leur activité anti-douleur. Ces exemples sont donnés à titre illustratif et ne sauraient être interprétés comme limitant la présente invention.

### Exemple I : Préparation, mesure de résistance aux glycanases et formulation des polymères biocompatibles.

### 1) Exemple de préparation.

Le médicament est obtenu par synthèse à partir d'un dextrane (Amersham Pharmacia T40 USP). Le procédé de synthèse comporte deux étapes de greffage chimique, une carboxyméthylation et une *O*-sulfonation.

### a) Protocole de carboxyméthylation

75 mL d'une solution de dextrane T40 (15 g, 92.5 mmol) dans de l'eau Milli-Q et 48 mL d'une solution contenant 29,6g (740 mmol) de soude sont préparés simultanément. La température des deux solutions est abaissée à 4°C avant de verser doucement la solution de soude dans la solution de dextrane. Le mélange réactionnel est agité pendant vingt minutes à 4°C. Après addition progressive de 30,6 g (323.8 mmol) d'acide chloroacétique, la température du mélange réactionnel est amenée à 50°C pendant cinquante minutes avant d'être neutralisé par de l'acide acétique (pH 7).

La solution obtenue après refroidissement est filtrée sur une membrane de 0,45µm (Millipore), le volume est porté à deux litres et du chlorure de sodium est additionné pour obtenir une concentration finale de 1M. Le sel de sodium du carboxyméthyldextrane est purifié par ultrafiltration à flux tangentiel sur une membrane de seuil de coupure de 10 000 Daltons (Pellicon^{®}, Millipore). Deux lavages successifs sont effectués à volume constant tout d'abord avec 2,5 l de chlorure de sodium 1M, puis avec 18 l d'eau Milli-Q. Le carboxyméthyldextrane est concentré puis le système d'ultrafiltration est rincé avec 250 ml d'eau Milli-Q.

La solution finale est déshydratée par lyophilisation.

### b) Protocole de sulfatation.

Le CMD, sous forme de sel de sodium, étant insoluble en milieu organique, il est nécessaire dans un premier temps, d'acidifier le polymère par percolation sur une résine échangeuse d'ions (Amberlite IR 120) pour générer un CMDH⁺. Cette forme en solution est ensuite lyophilisée. Le produit se présente alors sous forme de fibres cotonneuses de couleur blanche.

Le CMDH⁺ (6 g, 32 mmol) est mis en solution dans un mélange de FA (45 mL), de DMF (180 mL) et de 2-méthyl-2-buténe (45 mL) à 30°C. Le complexe de trioxyde de soufre, SO₃ / DMF (24.3 g, 160 mmol) est additionné à cette solution. On laisse réagir deux heures à 30°C sous agitation. Le mélange réactionnel est neutralisé par de l'hydrogénocarbonate de sodium (NaHCO₃, 5%) jusqu'à pH = 7.

Après filtration sur une membrane de 0,45µm (Millipore), le volume est porté à deux litres et du chlorure de sodium est additionné pour obtenir une concentration finale de 1M. Le sel de sodium du carboxyméthyldextrane sulfate est purifié par ultrafiltration à flux tangentiel sur une membrane de seuil de coupure de 10 000 Daltons (Pellicon^{®}, Millipore). Deux lavages successifs sont effectués à volume constant tout d'abord avec 5 l de chlorure de sodium 1M puis avec 18 l d'eau Milli-Q. Le carboxyméthyldextrane sulfate est concentré, puis le système d'ultrafiltration est rincé avec 250 ml d'eau Milli-Q.

La solution finale est déshydratée par lyophilisation.

Le degré de substitution (ds) des groupes carboxyméthyle (dsCM) se définit comme étant le degré de substitution par unité de glucose des groupes carboxyméthyle et le dsS se définit comme le degré de substitution par unité de glucose des groupes sulfates. Le produit OTD 70 obtenu répond aux critères analytiques suivants :

| | |
|---|---|
| dsCM : | 0,25 à 0,75 |
| dsS : | 0,80 à 1,30 |

Dans les exemples ci-dessous, les polymères biocompatibles ont été préparés en solution de sérum physiologique.

### 2) Mesure de résistance à la digestion par les glycanases.

Est présentée ci-après une étude comparative des effets des glycanases sur les RGTAs et sur les substrats naturels que sont les glycosaminoglycanes de mammifères. Les glycanases utilisées provenant de chez Sigma (US) sont les chondroïtinases ABC, l'héparitinase 1 et l'héparinase. La Hyaluronidase est de chez Seikagaku (Japon). Toutes ces enzymes sont d'origine bactérienne.

Les deux premières sont mises en solution par 2 unités dans 100 µl de tampon acétate 100 mM à pH 7,4, alors que les héparitinases et héparinases sont à 50 milliunités par ml de tampon 10 mM Sodium Acétate pH 7,0, avec 0,5 mM de calcium acétate et 100 µg/ml de BSA.

Les GAG substrats de référence ont été de la chondroïtine sulfate A (CSA) d'origine bovine, la chondroïtine B (CSB) d'origine porcine, la chondroïtine sulfate C (CSC) de cartilage de requin et de l'héparine (HS) d'origine bovine. Ces GAG ont été mis en solution à 200 mg/ml dans le tampon acétate à 100 mM à pH 7,4. Les RGTA ont été mis en solution à 400 mg/ml.

Les conditions d'incubation ont été de 6 heures à 37°C et la formation de fragments après digestion par ces enzymes, a été réalisée après un marquage des GAG du milieu réactionnel par de l'acide anthracique (Fluka) ; une séparation par HPLC est réalisée à l'aide d'une colonne d'exclusion TSK 3000 PWXKL N°3 PWX04B333 dans une phase mobile de tampon acétate en 1M NaCl sous un débit de 1ml/min et un temps d'élution de 120 min. La détection est réalisée à l'aide d'un détecteur Infra rouge réglé à une longueur d'ion d'excitation à 310 nm et d'émission à 410 nm, l'analyse est réalisée au travers d'un logiciel Eurochrom. es mesures des activités de dégradation des différentes enzymes sont calculées par intégration des pics séparés par chromatographie en fonction du temps de la réaction. La surface du pic du GAG ou RGTA au temps zéro étant le référentiel de 0% de dégradation.

Le tableau 1 ci-dessous représente le pourcentage de dégradation après 6 heures à 37°C.

**Tableau 1**

| Glycanase | | | | |
|---|---|---|---|---|
| | ChA BC | Hyaluronidase | Héparinase | héparitinase |
| CSA | 100 | | 7,2 | 26,2 |
| CSB | 100 | | 12 | 27 |
| CSC | 100 | | 20 | 25 |
| Héparine | 100 | | 100 | 100 |
| Acide Hyaluronique | | 100 | 100 | 100 |
| RGTAOTD120 | 20 | 25 | 23 | 23 |
| RGTAOTD70 | 20 | 22 | 27 | 15 |
| RGTA-E87 | 10 | 10 | 15 | 15 |
| RGTA-G-36 | 5 | 5 | 5 | 5 |
| RGTA-E82 | 5 | 5 | 5 | 5 |
| RGTA-21 | 5 | 5 | 5 | 5 |
| RGTA-E61 | 5 | 5 | 5 | 5 |
| RGTA-E57 | ND | DN | 5 | ND |
| RGTA-MP4 | ND | ND | 5 | ND |
| RGTA-FU | ND | ND | 5 | ND |
| RGTA-LG | ND | ND | 5 | ND |
| RCTA-XA | ND | ND | 5S | ND |

| | | | | |
|---|---|---|---|---|
| Exemples de référence: RGTA-MP4, RGTA-FU, RGTA-LG. | | | | |

### 6) Formulations.

Les préparations utilisées par administration locale sont à des concentrations de 100 microgrammes de polymères biocompatibles par millilitre de solution ou pommade (volume final) sauf indication contraire. Les modes d'application locale ont été par dépôt direct de gouttes sur la lésion douloureuse, instillation, absorption par voie buccale, contact par rinçage de bouche, ingestion; par aérosol, inhalation, par imprégnation d'une gaze ou d'un pansement et application de la gaze ou du pansement imprégné sur la zone douloureuse, par injection sous-cutanée au voisinage de la zone douloureuse.

En formulation de pommade, les compositions suivantes ont été réalisées à l'aide de gel de carboxyméthylcellulose de sodium (Aqualon) à 4,5% complément à 100% ou encore en gel neutre d'hydroxypropylcellulose (Kucel d'aqualon type 99MF EP) à 3% complément à 100% ou encore de la pommade d'hydricérine à 33% (H/L (phase Hydrophile dispersée dans une phase Lipophile, comme l'excipient de chez Roc ® contenant de la vaseline, de l'huile de paraffine, des glycérides, des éthers de polyoxylène et de la cérisine) complément à 100%.

Une formulation particulièrement efficace pour les applications locales sur des peaux fermées consiste à utiliser des gels antibactériens commercialisés pour le lavage des mains sans eaux «gels antibactérien sans rincage». Plusieurs de ces produits à base de polyéthylène glycol, alcool, glycérine sont faciles à se procurer (par exemple Assanis® de chez Blue skin®) dans lesquels le biopolymère est stable et mis à une concentration finale entre 0,1 et 1000 microgrammes par ml, avec une concentration préférée de 100 microgrammes).

Pour les solutions injectables par voix systémique, le RGTA était en solution de sérum physiologique à 1,5mg/kg, sauf autrement spécifié.

Le tableau 2 ci-après précise les types de polymères biocompatibles (poids moléculaires, types et degrés de substitution) utilisés dans les différents exemples.

**Tableau 2**

| Exemple^{a} | Polymères biocompatibles | Structure de départ | Structure | | | | |
|---|---|---|---|---|---|---|---|
| | | | Poids mol.^{b} | Z | Substitution Degré | | |
| | | | | | X=CM | Y=SO3 | Z |
| | | | | | x | y | z |
| 1,3 | RGTAOTD2 01 | Dextran | 2 | - | 0,4-0,5 | 0,7-1 | - |
| | RGTAOTD2 02 | Dextran | 10 | - | 0,4-0,5 | 0,7-1 | |
| Tous les exemples | RGT A OTD70 | Dextran | 40 | - | 0,4-0,5 | 0,7-1 | - |
| 3 | RGTAE-87 | Dextran | 70 | - | 0,39 | 1,10 | - |
| 3 | RGTAC-17 | Dextran | 200 | - | 0,48 | 1,15 | - |
| 3 | RGTAC-6 | Dextran | 500 | - | 0,43 | 1,30 | - |
| 3 | RGTAC -21 | Dextran | 2000 | - | 0,52 | 0,93 | - |
| 1, 3, 5, 6, 7, 8 | RGTAE-97 | Dextran | 40 à 2000 | - | 0,38 | 1,17 | - |
| 1, 3, 5, 8, 10 | RGTAG-36 | Dextran | 10 | Phenylalanine methyl ester | 0,59 | 0,83 | 0,22 |
| 3, 5, 6, 12 | RGTAC-27 | Dextran | 40 | Phenylalanine methyl ester | 0,76 | 0,81 | 0,18 |
| 3, 13 | RGTAC 29 | Dextran | 200 | Phenylalanine methyl ester | 0,57 | 0,87 | 0,18 |
| 3, 13 | RGTAC -31 | Dextran | 500 | Phenylalanine methyl ester | 0,46 | 0,88 | 0,27 |
| 3, 6 | RGTAC -32 | Dextran | 2000 | Phenylalanine methyl ester | 0,47 | 0,88 | 0,22 |
| Tous les exemples | RGTAE -82 | Dextran | 40 | acetate | 0,4 | 1,00 | 0,18 |
| 1, 3, 5, 6, 10 | RGTA-21 | Dextran | 40 | benzylamine | 0,82 | 0,95 | 0,47 |
| 3, 11, 12, 13 | RGTAE -61 | Cellulose | -90 | - | 0,39 | 1,13 | - |
| 3 | RGTAE -57 | β-glucan | nd | - | 1,04 | 0,89 | - |
| 1, 3 | | Carboxy Methylcellulose | | | | | |
| 1, 3, 5, 6 | | Glucuronane | 200 | acetate | 1 | -0.85 | 0,70 |
| 3 | | glucoglucuronane | 550 | | 0,5 | 0,9 | |
| 3, 5, 6 | RGTA MP4 | Copolymer Acide Malique | 6300 | S-Butyl | 0,65 | 0,25 | 0,10 |
| 3, | RGTAFU | Fucane | nd | | 0,7 | 1,8 | |
| 3, 5, 6, | RGTALG | Alginate | nd | | 1 | 0,7 | |
| 3, 5, 6 | RGTAXA | Xanthane | nd | | 0,7 | 1,1 | |
| 3, 5, 6 | Control | Polypensosulfate | 4 à 6000 | | | | |

| | | | | | | | |
|---|---|---|---|---|---|---|---|
| a : exemple pour indiquer le numéro du ou des exemples dans lequel (lesquels) ledit polymère biocompatible est utilisé. b : poids mol. Pour indiquer le poids moléculaire du squelette initial servant à la synthèse du polymère biocompatible exprimé en kDa. nd pour non déterminé. Exemples de référence: RGTA-MP4, RGTA-FU, RGTA-LG, RGTA-AXA. | | | | | | | |

### Exemple II : Effets anti-douleurs des polymères biocompatibles.

### 1) Exemple 1 : Brûlures superficielles de la peau : + après exposition au soleil :

Des brûlures du premier degré induites par une exposition prolongée aux radiations du soleil ont été traitées par des compositions contenant au moins un polymère biocompatible. Les brûlures se situaient au niveau du visage, du dos, du ventre, des bras, des jambes et/ou des pieds. Pour les personnes exposées d'une manière bilatérale, un seul des deux côtés ou membres exposés a été traité en gardant comme témoin l'autre côté ou membre. Les personnes dans le cadre de cette étude sont des adultes ou des enfants des deux sexes et d'âges allant de 5 ans à 60 ans. L'exposition au soleil se manifestait par une rougeur et hypersensibilité à la douleur avec, dans certains cas, un début de formation de cloques. Toutes les personnes avaient des atteintes après des expositions de durées variables, parfois d'une heure seulement, parfois de plusieurs heures par exemple au soleil des Bahamas entre 11H et 17H dans la première période d'Avril. Il s'agissait pour tous d'une première exposition après la période hivernale. Le soir vers 21H, quinze personnes souffrant de douleurs moyennes ou fortes mais permanentes ont été traitées par application locale d'une solution de polymères biocompatibles à 100 microgrammes par ml dans du sérum physiologique dans un excipient de méthylcellulose afin de donner une légère consistance pâteuse à la solution et faciliter son application et son adhésion sur la peau. Les volumes utilisés étaient juste suffisants pour recouvrir la surface de la zone à traiter et l'application consistait à un très léger passage du creux de la main afin d'étaler la solution pâteuse de polymères biocompatibles.

Dans tous les cas, ces personnes ont constaté un soulagement très net dans un délai de 10 à 30 minutes, voire une disparition de la sensation de douleur du côté traité au polymère biocompatible (dos, ventre, visage ou membre) alors que le côté controlatéral était toujours douloureux et restait très sensible au toucher. La douleur n'était pas réapparue le lendemain matin sur les régions traitées alors qu'elle était toujours persistante dans les zones brûlées non traitées. Selon l'importance de la brûlure et selon les cas après une vingtaine d'heures, la douleur était réapparue et le traitement était alors répété. Dans ces exemples, la douleur avait disparu après 48H dans tous les cas car il s'agissait de brûlures encore superficielles.

Des observations analogues ont été enregistrées avec application de polymère biocompatible seulement en sérum physiologique, aux mêmes concentrations que décrites ci-dessus.

Dans d'autres exemples similaires, le polymère biocompatible avait également un effet de soulagement de la douleur même si son application était réalisée sur une peau préalablement traitée avec des produits contenant des graisses comme de la vaseline ou de la Biaffine (Medix). La seule contrainte était alors de veiller à bien étaler la solution aqueuse de polymère biocompatible de manière à ce qu'elle ne coule pas à côté de la zone à traiter lors de l'application sur la peau. Ces mêmes effets ont été observés même si le polymère biocompatible a été appliqué plus tardivement après la brûlure.

Les effets du polymère biocompatible sont indépendants de ceux de l'aspirine ou du paracétamol ou d'autres agents anti-inflammatoires ou antalgiques souvent associés au soulagement de la douleur comme c'est le cas après une brûlure par le soleil. Ainsi, les effets différentiels antalgiques du polymère biocompatible se démarquent et s'ajoutent à ceux de ces agents et lorsqu'un seul côté brûlé est traité avec un polymère biocompatible chez une personne qui a absorbé (par voie orale) de l'aspirine ou du paracétamol (jusqu'à 1 gramme toutes les 6 heures), le côté brûlé traité avec un polymère biocompatible est plus soulagé que le controlatéral. La même expérience a été reproduite sur une dizaine d'autres personnes et chaque fois avec le même résultat.

### + après traitement au laser :

Des effets de soulagement ont été observés après applications du polymère biocompatible par vaporisation sur des peaux traitées au laser dans un protocole de «resurfacing». Le polymère était alors en solution dans du sérum physiologique à la concentration de 100 microgrammes par ml.

### 2) Exemple 2 : Effet antidouleur du polymère biocompatible sur les brûlures cutanées profondes.

Plusieurs cas de brûlures profondes ont bénéficié d'un traitement avec un polymère biocompatible et chaque fois un soulagement important voire une disparition de la sensation de la douleur ont été observés dans les dizaines de minutes suivant l'application et pour une durée de plusieurs heures.

Ainsi, un homme de 55 ans a, vers 21 heures, été brûlé à la main droite par un produit dérivé de produits lourds de pétrole et largement utilisé pour initier et activer les feux de barbecue. La brûlure a été diagnostiquée par deux médecins successivement le soir même et le lendemain matin comme étant du deuxième degré profond sur une surface représentant principalement environ la moitié de celle de la face externe de la main avec le pouce, l'index et le majeur et quelques zones de la paume allant jusqu'au poignet.

La douleur était extrêmement vive et le blessé devait, selon l'avis d'un chirurgien spécialiste des brûlures et qui était témoin de l'accident, être conduit à l'hôpital et traité par des analgésiques forts (Diantalvic ou morphine), suggérant l'éventualité d'une greffe de peau pour permettre de récupérer une pleine intégrité tissulaire. Environ deux heures après l'accident, une solution de polymère biocompatible à 100 Microg/ml dans du sérum physiologique imbibée sur des gazes stériles a été déposée sur les parties brûlées. Ces gazes ont ensuite été recouvertes par du tulle gras lui-même imprégné de vaseline.

Dans le quart d'heure qui a suivi l'application du polymère biocompatible, la douleur était devenue tout à fait tolérable et le suffisamment atténuée pour que le brûlé puisse s'endormir sans problème et se réveiller avec la sensation d'une disparition presque totale du sentiment de douleur. Le lendemain, le changement de pansement sur le même principe que la veille a encore augmenté la sensation de bien-être.

L'effet de soulagement et même de suppression totale de la douleur a persisté tout au long du traitement consistant uniquement à appliquer pendant une dizaine de jours sur les lésions une gaze imbibée de la solution de polymère biocompatible.

### 3) Exemple 3 : Effet antidouleur du polymère biocompatible chez le rat sur des brûlures profondes du deuxième degré.

Le modèle retenu consiste en des brûlures pratiquées sur la face dorsale de rats sans poils (rats hairless). Le modèle a été standardisé par des études préliminaires qui ont établi la reproductibilité du modèle d'étude. A l'origine, ce modèle a visé à évaluer l'effet du traitement par un polymère biocompatible sur la cicatrisation des brûlures et plus précisément au niveau de la ré-épidermisation, de l'angiogenèse et du remodelage du derme. Dans l'étude présentée ci-dessous, seuls sont rapportés les effets des polymères biocompatibles comme agents antidouleur observés au travers des critères notés de 1 à 5 mentionnés ci-dessus.

### a) Présentation du modèle.

Le modèle consiste en une brûlure obtenue par application pendant 4 secondes d'un cylindre de laiton de 2 cm de diamètre porté à 100°C dans un bain-marie bouillonnant. La nécrose de coagulation provoquée par l'application sur la peau du rat hairless, de ce cylindre n'est pas totalement homogène d'un point à un autre de la brûlure. Lors de l'examen histologique à trois jours, trois zones peuvent être distinguées en allant de la périphérie vers le centre de la brûlure, zones au niveau desquelles l'épaisseur progressive de la nécrose de coagulation est responsable de brûlures de profondeurs croissantes. Ces trois zones correspondent à des degrés différents de brûlure.

Ainsi, la zone périphérique de la brûlure où la nécrose de coagulation affecte seulement la couche cornée de l'épiderme correspond à une brûlure du premier degré. La zone intermédiaire de la brûlure où la nécrose de coagulation s'étend de l'épiderme jusqu'à la couche granuleuse ou à la partie superficielle du corps muqueux de Malpighi. Cette nécrose de coagulation affecte également l'épithélium des infundibulums pilaires sur une épaisseur équivalente. Il s'agit d'une brûlure superficielle du deuxième degré. Enfin la zone centrale de la brûlure où l'épiderme est nécrosé sur toute sa hauteur, représente de 50 à 60% de la surface totale brûlée, et correspond à une brûlure du deuxième degré profond.

Ainsi suivant les zones, la nécrose respecte ou non la basale épidermique et touche ou non la partie attenante du derme papillaire. Il s'agit d'une brûlure profonde du deuxième degré ou d'une brûlure superficielle intermédiaire. Des lésions de nécrose identiques à celles de l'épiderme s'observent au niveau de l'épithélium des infundibulums pilaires et s'étendent plus ou moins complètement jusqu'au contenu des glandes sébacées.

### b) Protocole expérimental.

Quatorze rats mâles hairless, pesant entre 280 et 300 grammes, ont été répartis par tirage au sort en deux groupes de 7 rats. Après anesthésie des rats selon les protocoles réglementaires de l'expérimentation animale, les brûlures sont réalisées selon le protocole décrit ci-dessus.

Cinq minutes après la réalisation des brûlures, les animaux des groupes traités (G+) et témoin (G-) reçoivent une application topique de 1 millilitre de sérum physiologique contenant ou non 100 microgrammes par millilitre de polymère biocompatible, imbibé sur une compresse stérile. Quelques minutes après, chaque animal reçoit une injection intramusculaire (IM) de sérum physiologique contenant ou non du polymère biocompatible (1,5 milligramme Kilo) sous un volume de 300 microlitres. Les animaux des groupes G+ reçoivent ensuite une injection hebdomadaire par IM de la même dose de polymère biocompatible et ceux des groupes G- une injection de sérum physiologique. Les pansements occlusifs sont constitués d'une couche de tulle gras, surmonté d'un film de Jelonet, Opsite (10 X 14 cm, Smith and Nephew). L'ensemble est recouvert par un sparadrap entourant tout l'abdomen de l'animal.

Les pansements sont changés tous les 2 jours la première semaine puis de façon hebdomadaire le premier mois. Les animaux gardés en cage individuelle n'ont plus de pansement un mois après l'opération.

### c) Mesure de la souffrance des animaux.

A J1, J3, J5 et J7, lors des changements de pansement, on note une différence dans le comportement des animaux traités par un polymère biocompatible (+) avec les animaux non traités (-). Les animaux traités sont beaucoup plus calmes et sont manipulés sans se débattre par l'expérimentateur et n'émettent pas de cris. Pour éviter des interférences, les changements de pansements sont réalisés dans une pièce séparée de l'endroit où les animaux sont gardés et les cages individuelles sont retirées au hasard (rats traités ou non traités par un polymère biocompatible étant changés de pansement sans ordre pré-établi). L'expérimentateur, qui n'est pas informé du traitement par un polymère biocompatible des animaux, protocole en double aveugle, note de 1 à 5 les paramètres suivants:
- Paramètre (a) : Facilité avec laquelle se laisse prendre le rat (1 étant le plus facile) ;
- Paramètre (b) : Facilité avec laquelle il se laisse enlever le pansement ;
- Paramètre (c) : Réaction à la palpation (toucher) de la zone brûlée ;

Le toucher est réalisé par application du tube en laiton utilisé précédemment pour la brûlure, mais à température ambiante, appliqué sans pression sur le centre de la plaie, maintenu en position droite avec la main, pendant 5 secondes.
- Paramètre (d) : Réaction à la palpation en zone périphérique à la plaie;

Après l'expérience du toucher, la réaction de l'animal à des mouvements de peau est mesurée. Ceci est réalisé en tirant la peau saine prise entre le pouce et l'index de chaque main de l'expérimentateur placée de chaque côté à environ 3 cm du centre initial de la brûlure et par 4 mouvements lents de va et vient d'une amplitude de 2 cm selon un axe perpendiculaire à la colonne vertébrale, l'animal étant maintenu par un deuxième expérimentateur au niveau de la base du cou et des pattes arrières.
- Paramètre (e) : Facilité avec laquelle le rat se laisse traiter pour la détersion de la plaie et le remplacement du pansement ;
- Paramètre (f) : Si les animaux émettaient des signes sonores de souffrance à chacune de ces étapes.

Le tableau 3 ci-après résume les résultats expérimentaux obtenus.

**Tableau 3**

| Jours | J1 | | J3 | | J5 | | J7 | |
|---|---|---|---|---|---|---|---|---|
| Traitement par du RGTA | - | + | - | + | - | + | - | + |
| Paramètre a | 5 | 2 | 5 | 1 | 2 | 1 | 2 | 1 |
| Paramètre b | 5 | 3 | 3 | 2 | 3 | 1 | 3 | 1 |
| Paramètre c | 5 | 3 | 5 | 2 | 5 | 1 | 3 | 1 |
| Paramètre d | 5 | 3 | 5 | 2 | 5 | 1 | 4 | 1 |
| Paramètre e | 5 | 2 | 4 | 2 | 4 | 1 | 4 | 1 |
| Paramètre f | 5 | 2 | 5 | 1 | 3 | 1 | 3 | 1 |

| | | | | | | | | |
|---|---|---|---|---|---|---|---|---|
| Notation de 1 à 5 par ordre croissant d'appréciation des critères | | | | | | | | |

Il est à noter que ces effets anti-douleurs du olymère biocompatible sont observés bien avant même les effets sur la cicatrisation qui n'apparaissent dans ce modèle que 8 à 10 jours après la brûlure, temps nécessaire pour éliminer les tissus nécrotiques.

### 4) Exemple 4: Effet antidouleur chez l'homme normal atteint d'ulcères cutanés sévères de toutes origines et de neuropathies périphériques, notamment chez les diabétiques et les alcooliques.

Dix cas d'ulcères cutanés chez l'homme ont été traités. Il s'agit de cas compassionnels, présentant tous une artérite de stade IV, pour laquelle aucun traitement n'avait eu d'effet et pour lesquels l'amputation du membre était inévitable et prévue comme seule alternative à un pronostic morbide.

Deux protocoles ont été utilisés:
a) le premier a consisté à déposer sur la plaie après détersion, une gaze stérile (10 X 10) préalablement imbibée par 5 ml de polymère biocompatible à 100 microgrammes par ml dans du sérum physiologique injectable chez l'homme et la laisser sur la plaie pendant 10 minutes. La gaze est enlevée et le pansement est fait de façon traditionnelle. Les 8 premiers cas ci-dessous ont été traités selon ce protocole.
   Le premier cas est un homme de 75 ans ulcéré à la maléole interne, sur une très grande surface, rétif à tout traitement depuis deux ans, et sous morphine (Skenan, 2 fois 90 mg/jour). Dès le premier jour de traitement, le malade a senti un soulagement de la douleur qui a complètement disparue après 14 jours. Bien évidemment le malade a arrêté de prendre son médicament antalgique. Il est à noter que l'effet antidouleur du polymère biocompatible est apparu bien avant l'effet sur la cicatrisation, qui s'est étalé sur une période de plus de deux mois.
   Le deuxième cas est un homme de 69 ans, diabétique et dont la face dorsale du pied est ulcérée si profondément que les tendons sont à nu. L'ulcère est rétif à tout traitement depuis plus de 6 mois et ce malade est sous antalgique morphinique. Le traitement par le polymère biocompatible a permis un soulagement tel de la douleur qu'après 14 jours, le malade a arrêté de prendre ses antalgiques.
   Le troisième cas est une femme de 50 ans, déjà amputée et dont le moignon est ulcéré. L'ulcération évolue et le démembrement complet du moignon jusqu'à la hanche est programmé. Cette femme est sous morphine et benzodiazépines. L'application du polymère biocompatible a permis un soulagement de la douleur tel qu'après deux semaines le malade a arrêté de prendre et la morphine et les benzodiazépines.
   Les mêmes effets antidouleur du polymère biocompatible ont été observés dans les cinq autres cas traités.
b) Le deuxième protocole d'administration du biopolymère a été oral et a consisté à donner à boire tous les matins à jeun une solution de 70 ml du polymère dans de l'eau et à des concentrations qui, selon les patients, allaient de 0,01 à 10 mg/kg (poids du patient). Tous les patients traités (3 cas) ont été soulagés de leur douleur et ceci très nettement après 15 jours de traitement. L'effet antidouleur étant apparu plus rapidement aux fortes doses, mais obtenu à terme avec les doses les plus faibles.
La majorité des cas traités ci-dessus étaient diabétiques. Il est ressorti de cette étude qu'un traitement par voie orale permettait aussi de soulager les neuropathies notamment des membres inférieurs chez les diabétiques et/ou les alcooliques et même chez quelques malades atteints de sida sans que ces personnes ne souffrent d'ulcérations. L'effet de soulagement de la douleur a été observé dans tous ces cas.

### 5) Exemple 5 : Effet antidouleur du polymère biocompatible sur la douleur induite par les boutons de fièvre.

Les herpès labiaux et génitaux sont une infection courante qui se traduit par la formation de boutons au niveau des lèvres (également appelés boutons de fièvre) et muqueuses. Ils évoluent en gonflant, devenant rapidement rouge et régressent en provoquant des fissures superficielles. Ces différentes étapes correspondent à des phases de développement du virus et se traduisent par une douleur croissante avant l'éclatement puis après la formation de la fissure jusqu'à la régression.

L'application locale de polymère biocompatible par simple étalement avec le doigt sur le bouton soulage la douleur et que ce soulagement est aussi perçu lors de la phase d'éclatement et de fissuration.

L'effet du polymère biocompatible sur la cicatrisation de cette fissure était prévisible par les effets déjà décrits du polymère biocompatible sur la cicatrisation des lésions cutanées, mais il n'était pas prévisible que l'effet du polymère biocompatible soit perçu sur la douleur dès la formation du bouton, pendant sa croissance et au moment de son éclosion.

### 6) Exemple 6 : Effet antidouleur du polymère biocompatible sur la douleur induite par les gerçures sur les doigts.

La formation de gerçure est fréquente et touche toutes les populations des deux sexes et de tout âge. Ces gerçures provoquent une douleur sourde augmentée dès que les doigts sont exposés à la sécheresse, à l'humidité et/ou au froid.

Il a été observé essentiellement sur des populations de femmes d'âge compris entre 40 et 80 ans qui souffrent de gerçures plusieurs mois de l'année qu'une seule application locale d'une solution de polymère biocompatible induit un soulagement de la douleur. Ces personnes ont toutes utilisé sans succès des traitements commerciaux à base de graisse protectrice ou de crème type Neutrogena.

Le soulagement est perceptible dès la première demi-heure et en général dure car il est accompagné par une fermeture rapide des micro-lésions cutanées provoquant la gerçure dans les 24 à 48H suivant sans qu'il soit besoin d'effectuer une autre application de polymère biocompatible.

Plusieurs dizaines de personnes ont ainsi pu être traitées avec chaque fois le même succès. Le polymère biocompatible a dans tous les cas traité la douleur et la gêne occasionnées par la gerçure dans un premier temps et les microfissures cutanées dans un deuxième temps.

Souvent un effet synergique du polymère biocompatible avec des crèmes antibiotiques (auréomycine ou néomycine à 2%) a souvent été observé.

### 7) Exemple 7 : Effet antidouleur du polymère biocompatible sur la douleur et/ou les démangeaisons induites par une hyperkéritinisation de la peau ou par le psoriasis ou par de l'eczémas et par un zona

Un effet d'apaisement de l'irritation cutanée ou des démangeaisons provoquées par des dermites de diverses origines a été observé dans différents cas suite à un traitement par des polymères biocompatibles.

Ainsi, un dentiste dont les mains étaient devenues hyperkératinisées à la suite d'une dermite rétive à tout traitement souffrait au point de plus pouvoir exercer son métier a été soulagé par l'application de polymère biocompatible en solution ou en pommade (par exemple en gel chargé de carboxymethylcellulose méthyl cellulose) alors que seuls des corticoïdes en pommade avaient permis de le soulager mais pas avec la même efficacité et surtout le traitement n'avait pas pu être prolongé. Un traitement quotidien de 15 jours par le polymère biocompatible suivi d'une application hebdomadaire a suffi à régler durablement ce problème.

Il en est de même du soulagement apporté par un badigeonnage de crevasses qui se forment sous les pieds dans les zones fortement kératinisés. Ces crevasses peuvent être très douloureuses. L'application de polymère biocompatible apporte un soulagement de la douleur et cet effet a été observé sur plusieurs personnes. Un des effets les plus marquants du polymère biocompatible de l'invention est de calmer les démangeaisons que celles ci soient locales ou générales. Ainsi plusieurs cas de démangeaison d'origine multiples locale ou systémique ont été calmés par application locale ou absorption per os du polymère de l'invention

### 8) Exemple 8 : Effet de soulagement et antidouleur du polymère biocompatible sur les irritations des muqueuses ou de la peau ainsi que l'effet antidouleur après opération chirurgicale.

L'application locale du polymère biocompatible sur des muqueuses et/ou peau irritées ou encore des cicatrices fermées soulage la douleur, les démangeaisons et le sentiment de brûlure causés par ces irritations ou sans cause connue. Cette observation a été faite sur un grand nombre de cas, que l'irritation soit d'origine mécanique (frottement des mouchoirs sur le nez, de la langue sur les lèvres etc...) et/ou soit due à un défaut de sécrétion de lubrifiant par les glandes appropriées (lacrymales, salivaires, vaginales etc...) ou causée par d'autres facteurs comme un excès de sécrétion dans le cas des rhinites. Le soulagement de la douleur et des démangeaisons sur les cicatrices fermées souvent encore turgescentes était très net après application locale du polymère dans les formulations en gel ou en crème. Nous avons été aussi surpris de voir que ces cicatrices, au fur et à mesure du traitement se réduisaient en épaisseur et devenaient beaucoup plus fines, avec de manière concomitante une rougeur fortement diminuée. Ces deux effets inattendus ont été étudiés d'une manière comparative sur un même individu sur des grandes cicatrices ou sur des cicatrices symétriques. Ainsi sur le ventre des cicatrices allant de part et d'autre du nombril (après une colonectomie) ou au niveau du sternum (après une chirurgie cardiaque) la cicatrice longiligne a été divisée en trois parties égales par des marques dessinées sur un cache en carton couvrant toute la peau autour d'une fente laissant visible et accessible la cicatrice et ses bords sur environ 2 cm de largeur. Le traitement au polymère biocompatible a été réalisé par application avec le doigt du gel contenant soit du polymère biocompatible de l'invention soit le gel seul sur soit la partie supérieure soit la partie inférieure de la cicatrice pendant 15 jours. La douleur était fortement diminuée jusqu'à avoir disparue dans certain cas dans la partie traitée au polymère alors qu'elle restait nette dans la zone non traitée. De même la cicatrice avait largement été réduite dans la zone traitée au polymère de l'invention et sa rougeur également diminuée comparativement à la zone traité par le véhicule. Une observation similaire et encore plus nette a été faite dans le cas des cicatrices situées sur des zones symétriques comme celles provenant d'une plastie mammaire bilatérale ou des deux cicatrices derrière les deux oreilles réalisées lors d'un lifting.

La Demanderesse a naturellement recherché si cet effet antidouleur était observé après une chirurgie. Il a pu être proposé une administration orale du polymère au réveil de l'anesthésie puis biquotidienne, matin et soir de 2 fois 25 ml de la solution dans de l'eau du polymère à des concentrations de 10 microgrammes à 10 milligrammes d'un des polymères biocompatible. L'effet antidouleur a été observé après différentes chirurgies comme des chirurgies orthopédiques (installation de prothèse de la hanche, réparation de défaut osseux sur des tibias, chirurgie d'implants dentaires, chirurgie viscérale: appendicites et colonectomie).

### 9) Exemple 9: Effets antidouleur du polymère biocompatible sur les lésions cutanées induites par radiothérapie.

Le traitement des cancers par radiothérapie provoque des brûlures et des micro-lésions cutanées douloureuses dans la zone irradiée. Plusieurs patients ont été traités parfois avant irradiation mais toujours après par du polymère biocompatible déposé en gouttes, en vaporisation (spray) ou en gel sur la zone irradiée. Dans tous les cas, la douleur a été soulagée dans l'heure qui a suivi l'irradiation. L'application de polymère biocompatible en pommade avant l'irradiation a également été efficace pour soulager la douleur d'une manière préventive.

### 10) Exemple 10 : Effet antidouleur du polymère biocompatible sur les souffrances du tympan.

Sur un cas adulte (homme de 56 ans) souffrant d'une otite bilatérale et douloureuse des deux côtés, du polymère biocompatible a été instillé en gouttes sur un seul des tympans. L'effet antidouleur du polymère biocompatible n'a été ressenti que sur l'oreille traitée. La douleur dans l'oreille traitée étant revenue le lendemain, la deuxième instillation a été réalisée cette fois sur les deux tympans qui ont été tous les deux soulagés.

### 11) Exemple 11 : Effet antidouleur du polymère biocompatible sur les douleurs de la cornée.

Le modèle utilisé est celui de l'induction d'ulcères sur des cornées de lapins par application pendant quelques secondes d'un disque de 0,5mm de diamètre imbibé d'une solution de NaOH. Le jour de l'ulcération, les lapins anesthésiés sont traités par instillation de deux gouttes de polymère biocompatible en solution à 100 microgrammes par ml dans du sérum physiologique ou par instillation de deux gouttes de sérum physiologique seul (contrôle).

Le lendemain, l'effet analgésique de l'anesthésie étant disparu, les lapins montrent une sensibilité extrêmement différente selon qu'ils ont ou non reçu un traitement au polymère biocompatible. Les lapins traités se laissent ouvrir l'oeil traité sans cris, n'ont pas de photophobie visible. L'instillation de polymère biocompatible est reçue immédiatement comme un soulagement alors que l'instillation de sérum physiologique ne semble pas provoquer de soulagement chez la population témoin.

Ces effets observés chez le lapin ont depuis été confirmés chez l'homme. Ainsi, un effet de confort et de soulagement a-t-il été perçu chez les personnes portant des verres de contact et qui ressentaient une gêne et irritation manifestée par des picotements. L'instillation d'une goutte d'une solution de polymère biocompatible a immédiatement soulagé et permis de maintenir le port de ces lentilles alors que, sans polymère biocompatible, les lentilles devaient être enlevées. L'effet a été encore plus sensible chez des personnes traitées au laser dans le cadre d'une chirurgie correctrice. La gêne, parfois douloureuse pendant quelques jours et provoquée par cette chirurgie disparaît par simple instillation de une à deux gouttes de polymère biocompatible en solution à 100 microgrammes/ml, une à deux fois par jour.

### 12) Exemple 12 : Effet antidouleur du polymère biocompatible sur les brûlures des voies aériennes respiratoires.

Ce même soulagement a été observé après intoxications et brûlures pulmonaires dont les origines étaient l'inhalation de fumées provenant de feux ou de gaz toxiques. Dans ce cas, le polymère biocompatible a été inhalé après avoir été mis en aérosol.

Ainsi, lors d'un incendie provoqué par la combustion de bois, la forte fumée dégagée a provoqué une suffocation respiratoire et des brûlures pulmonaires douloureuses. L'inhalation de polymère biocompatible vaporisé en aérosol a provoqué un soulagement rapide de la douleur. Le polymère biocompatible était en solution aqueuse dans du sérum physiologique à 100 microgrammes/ml et l'aérosol réalisé à l'aide un tube en verre capillaire plongeant dans la solution et un système de pression gazeuse poussant le polymère biocompatible au travers ce capillaire. Ce même effet de soulagement de la douleur a été observé après inhalation accidentelle de vapeur de formol.

### 13) Exemple 13 : Effet antidouleur du polymère biocompatible sur les souffrances des voies aériennes respiratoires liées à l'asthme et aux rhinites.

Plusieurs personnes présentant des phénomènes d'allergies respiratoires de différentes origines (rhume des foins, allergie aux poils de chat ou de rat) ont été traitées par des aérosols obtenus en pulvérisant du polymère biocompatible, objet de la présente invention, directement dans les narines à l'aide d'un flacon pourvu d'un nébuliseur. Ce traitement administré en pleine crise provoque un soulagement rapide de la gêne.

Cette observation a été reproduite plusieurs fois sur les mêmes personnes et a apporté chaque fois un soulagement. Il a été observé que la fréquence des crises diminuait en fonction du nombre de traitements. De même, le traitement avec un polymère biocompatible selon l'invention diminue le sentiment de suffoquer et les quintes de toux provoquées par des expositions au tabac ou d'autres agents irritants.

Dans tous ces cas, les concentrations utilisées étaient de entre 1 et 100 microgrammes par ml, selon le polymère utilisé mis en solution étant, au départ, avant la formation d'aérosol, dans une solution saline aqueuse stérile.

Au fur et à mesure que la douleur puis la gène disparaissaient les autres symptômes aussi, et de manière surprenante en traitant la douleur la maladie a également été traité au moins pour une période de plusieurs mois. Il est à noter que dans quelques cas le traitement par inhalation de biopolymères de l'invention était réalisé en plus et sans modifier les thérapies et posologies en cours. Ainsi l'administration de produits de corticoïdes, de Bêta 2 antagonistes ne semble pas interférer avec l'action des biopolymères.

### 14) Exemple 14 : Effet antidouleur du polymère biocompatible sur les gênes et douleurs liées aux obstructions des bronches.

Les gênes et douleurs consécutives à l'obstruction des voies respiratoires résultants d'infections ou d'irritations ou obstruction des bronches comme dans la mucoviscidose dans des formes aiguës ou chroniques ont été soulagées par inhalation de polymères mis en aérosols mais également par absorption quotidienne par voie orale de solution allant de 0,01 à 10 mg/kg selon, les cas et types de polymère utilisé.

Au fur et à mesure du traitement de la douleur et des gènes respiratoires une amélioration des symptômes de la maladie a été observée. Dans certains cas, l'emphysème semblait être traité dans la mesure où la douleur n'est pas revenue et la fonction respiratoire s'est nettement améliorée.

### 15) Exemple 15 : Effets calmant de la toux du polymère biocompatible.

La fumée de cigarettes est une source fréquente d'irritation de la gorge, du nez et des bronches et peut entraîner une toux régulière ou des quintes de toux. Celles-ci peuvent devenir franchement douloureuses. L'absorption par voie orale avec gargarisme ou encore par nébulisation ou respiration en aérosol du polymère biocompatible a un effet de soulagement voir d'arrêt de la gêne provoquée par ces fumées rapides.

### 16) Exemple 16 : Effets antidouleur du polymère biocompatible sur les ulcérations d'estomac.

Les effets antidouleur sur les ulcérations de l'estomac ont été observés dans deux cas.

L'un était un homme de 45 ans africain et souffrant d'un ulcère à l'estomac diagnostiqué par endoscopie mais non traité (par antibiothérapie contre Helico Bacter) et suffisamment sévère pour que la douleur lui impose un régime sans alcool et sans aucun excitant gastrique (piments, café, etc.). Malgré ce régime, cet homme souffrait d'une sensation permanente de brûlure, particulièrement exacerbée aux heures des repas. L'ingurgitation par voie orale de 20 ml de polymère biocompatible en solution dans du sérum physiologique à 100 microgrammes par ml tous les trois jours a supprimé toutes douleurs et ce, dès le premier jour du traitement qui a duré trois semaines sans rechutes après 6 mois.

L'autre cas est un homme de 57 ans dont les sensations de brûlure à l'estomac sont régulièrement calmées dans les minutes suivant l'absorption d'une gorgée de 20 ml de polymère biocompatible.

### 17) Exemple 17 : Effet antidouleur du polymère biocompatible sur les douleurs diffuses au ventre.

Un homme de 55 ans de 70 kg présentant des douleurs diffuse au ventre avec une fréquence et intensité croissante puis disparaissant pour réapparaître quelques jours plus tard a été calmé efficacement à plusieurs reprises par une prise orale de 70ml de polymère biocompatible objet de la présente invention. Dans la demi-heure suivant la prise, la douleur avait disparu. Cet homme souffrait de colique néphrétique et l'effet antidouleur obtenu par cette prise orale n'était efficace que dans les premières phases de la colique. Lors de douleurs plus intenses, seule la morphine a pu être efficace. C'est cette observation qui a permis de prendre conscience d'une action antidouleur systémique du biopolymère par administration orale. Une femme de 55 ans, atteinte d'un cancer du pancréas inopérable a survécu sans devoir augmenter un taux basal de prise de morphine de 50 mg par jour jusqu'à un stade très tardif sans que la douleur ne devienne insupportable avec une prise orale et quotidienne de 100 microgrammes/kg du polymère en solution dans l'eau. Il ne semble pas exister la moindre interférence avec la morphine si ce n'est qu'un effet additif sur la douleur.

### 18) Exemple 18 : Effet antidouleur du polymère biocompatible sur les maux de tête.

L'effet du biopolymère sur les céphalées a été observé dans une dizaine de cas après une prise orale du produit selon les mêmes conditions que dans l'exemple 15. Là encore, la céphalée a pu être réduite fortement et parfois totalement. Dans deux des cas traités, les personnes, coutumières de fortes migraines et qui, en général, n'obtenaient pas de soulagement avec les produits antalgiques habituels (aspirine, paracétamol) n'ont plus eu mal.

### 19) Exemple 19 : Effets antidouleur du polymère biocompatible sur les aphtes, mucites, maux de gorges et sur et les douleurs dentaires.

L'application de polymère biocompatible soit directement sur des aphtes buccaux à l'aide d'un coton-tige imbibé de polymère biocompatible ou par simple bain de bouche provoque un soulagement radical de la douleur.

Il en est de même pour les douleurs au niveau des gencives provoquées par le déchaussement des dents. Dans ce cas, nous avons observé une meilleure efficacité après un brossage des dents et des zones sensibles imprégnée avec une brosse trempée dans une solution de polymère biocompatible.

L'effet antidouleur a été observé dans une série d'expérimentations dans un modèle de parodontites induites chez le hamster. Le modèle expérimental est tel que celui publié dans Escartin et al., «A new approach to treat tissue destruction in periodontitis with chemically modified dextran polymers», FASEB J., 2003, vol. 17, 636-643. Dans cette série d'expériences, les animaux traités par injection par voie intramusculaire de polymère biocompatible à 0,4 mg/kg ou à 1,5 mg/kg souffraient apparemment moins que ceux qui ne subissaient aucun traitement. La diminution de la douleur étant appréciée sur des critères comportementaux et à l'aspect des poils et se manifestant par une moins grande hypersensibilité des animaux aux stress que représentait l'approche de l'expérimentateur et l'agitation devant des bruits et surtout par une prise plus rapide des rations alimentaires, suggérant une gêne moindre des animaux traités avec un polymère biocompatible.

De même, une femme de 52 ans, souffrant de douleur de gorges et d'extinction de voie a été soulagée par des bains de bouches et gargarismes avec un polymère biocompatible.

L'effet marquant sur le soulagement des douleurs des mucites induites après chimio et radio thérapie a été observé après bain de bouche du polymère de l'invention. Plusieurs personnes atteintes de mucites de grade 2 et 3 ont été soulagées après bain de bouches des douleurs très pénibles résultant de la formation d'ulcères dans la bouche et la gorge. Un malade atteint de la maladie de Behçet. a également été fortement soulagé par bain de bouche matin et soir pendant les quelques jours qu'a duré la crise douloureuse. Le polymère était simplement en solution aqueuse à 100 microgrammes par ml.

### 20) Exemple 20 : Effets antidouleur du polymère biocompatible sur les tendons.

Plusieurs cas de douleurs tendineuses ont été traités par un polymère biocompatible administré par simple massage sur le site douloureux. Ainsi, les observations suivantes ont été réalisées.

Un adulte de 57 ans, gaucher et joueur de tennis avait une tendinite douloureuse au coude gauche et avait renoncé à pratiquer ce sport après avoir suivi plusieurs traitements dont plusieurs injections locales de corticoïdes à 6 mois d'intervalle et un arrêt total du tennis de deux ans. La douleur avait disparu, mais réapparaissait au moindre effort engageant ses tendons du coude. A l'issue d'une journée ayant fortement sollicité son coude, cet homme avait une douleur intense au coude. L'application locale de polymère biocompatible par massage avec une solution à 100 microgrammes par ml en sérum physiologique a en quelques dizaines de minutes complètement permis la disparition de cette douleur.

De même, il a été observé que l'injection locale ou l'application en pommade des polymères biocompatibles au niveau des zones douloureuses des tendons comme pour le coude ou encore le genou soulage rapidement et d'une manière durable la douleur. Ainsi, sur plusieurs tendinites du coude comme les "tennis elbow", ou les tendons ischémiés du genou comme dans la maladie de Osgood-Schlatter ou encore sur des souffrances des tendons des pieds comme le tendon d'Achille chez les sportifs, l'administration de polymère biocompatible induit des effets de soulagement rapide et importants des douleurs.

Le même traitement a été appliqué aux chevaux de courses souffrant de tendinites des zones articulaires des jambes. Ainsi une série d'injections dans la zone péritendineuse de quelques dizaines de microlitres de polymère biocompatible a permis de soulager la douleur et ce soulagement s'est manifesté par une absence de claudication après une quinzaine de jours. Le traitement a été poursuivi avec une fréquence hebdomadaire et une reprise de l'entraînement après deux mois a montré une absence de claudication, de douleur au toucher et une amélioration des performances des animaux.

### 21) Exemple 21 : Effets antidouleur du polymère biocompatible sur les arthroses ou arthrites.

Plusieurs cas de douleur émanant de problèmes arthrosiques ou inflammatoires au niveau de l'articulation du genou ont été soulagés par application de polymère biocompatible soit en solution en sérum physiologique à 100 microgrammes par ml soit aux mêmes concentrations en pommade faite avec du gel de carboxyméthylcellulose de sodium (Aqualon) à 4,5% complément à 100% ou un gel neutre d'hydroxypropylcellulose (Kucel d'aqualon type 99MF EP à 3% complément à 100%). Les personnes traitées étaient sous antalgique (3 comprimés par jour de Voltarène forme LP à 100). Un simple massage avec du polymère biocompatible a provoqué un soulagement après un quart d'heure pour une durée de deux à trois heures. Ce traitement répété a évité à ces personnes des prises de Voltarène. Après trois jours de ce traitement, les douleurs étaient ressenties de plus en plus rarement malgré un usage normal du genou. Enfin après une quinzaine de jours, l'application de polymère biocompatible une fois par jour était suffisante pour éviter toute douleur.

Des effets similaires ont été obtenus sur le long terme lorsque le polymère biocompatible a été donné en solution à boire. Les doses de 0,1 mg/kg à 10 mg/kg sont prises dans un volume d'eau de 50 à 100 ml tous les jours (de préférence le matin à jeun). Des soulagements ont été observés sur des personnes souffrant de douleur aux genoux depuis plusieurs années empêchant ainsi la pratique de sports et rendant la marche difficile et douloureuse. Après un traitement de deux mois, ces personnes n'ont plus éprouvé cette gêne et/ou douleur et ont pu à nouveau pratiquer leur sport. Elles n'ont pas observé d'effets adverses à ce traitement sur cette période.

### 22) Exemple 22 : Effets antidouleur du polymère biocompatible après une chirurgie orthopédique.

L'effet antidouleur du polymère biocompatible a été observé chez une femme de 50 ans qui venait de subir une opération de raidissement des os du pied. Il s'agissait d'une correction d'un Hallux Valgus et de deux autres doigts de pieds en insérant une tige métallique dans ces os afin de maintenir l'axe du doigt dans la bonne position. Cette opération déjà réalisée chez cette personne trois ans auparavant avait provoqué une très grande douleur pendant plus d'une semaine et une douleur encore importante pendant trois semaines. A la suite de la deuxième intervention, cette personne a reçu une injection intramusculaire de polymère biocompatible sur la base de 1,5 mg/kg aux jours 2, 4 et 8 post-opératoires. La douleur a disparu dans l'heure suivant la première injection pour revenir au 2^{ième} jour puis a de nouveau disparu dans l'heure qui a suivi la deuxième injection de polymère biocompatible le 4^{ième} jour pour ne revenir que le 7^{ième} jour. Elle a à nouveau disparu dans l'heure qui a suivi la réinjection au 8^{ième} jour et cette fois définitivement.

### 23) Exemple 23 Effet antidouleur du polymère biocompatible sur les neuropathies dégénératives.

Deux malades atteints de sclérose en plaque depuis plus de 20 ans souffraient régulièrement de douleurs articulaires, ventrales avec des spasmes douloureux périodiques. Ils avaient tous les deux un stade de la maladie assez proche avec la perte de l'usage d'une jambe.
La prise orale quotidienne d'environ 30 ml d'une solution aqueuse du polymère de l'invention a calmé pour partie leurs douleurs et notamment de la main pour l'un et articulaire et viscérale pour l'autre. La chose la plus surprenante a été de constater, après deux mois de prise orale du polymère de l'invention en solution aqueuse de 3 à 30 mg par jour, le retour à une légère récupération de la motricité de la jambe paralysée. Le soulèvement du talon, puis de la jambe puis la possibilité de l'avancer d'arrière en avant sans toucher le sol, puis de la soulever suffisamment pour pouvoir toucher le genoux ont été les signes objectifs de cette récupération. De même les souffrances ou neuropathies des jambes et mains chez des diabétiques type 2 ont été fortement soulagées après quelques semaines de prise orale de la solution aqueuse de polymère biocompatibles de l'invention.

### 24) Exemple 24 : Effet antidouleur du polymère biocompatible sur les cystites.

La douleur persistante de cystite rebelle à une antibiothérapie a été soulagée chez deux jeunes femmes par une prise quotidienne orale du polymère de l'invention, en addition d'une antibiothérapie.

Exemples de référence: Exemples 1-6, 9-24.

## Revendications

1. Composition pharmaceutique, dermatologique ou cosmétique pour son application comme médicament destiné à la prévention, le soulagement et/ou le traitement du prurit et/ou à la protection des tissus contre ceux-ci, ladite composition comprenant un polymère biocompatible de formule générale (I) suivante
AaXxYy (I)
dans laquelle :
A représente un monomère, choisis parmi le glucose,
X représente un groupement RCOOR',
Y représente un groupement O ou N-sulfonate fixé sur A et répondant à l'une des formules suivante -ROSO3R', -RNSO3R' dans lesquelles :
R représente une chaîne hydrocarbonée aliphatique, éventuellement ramifiée et/ou insaturée et qui peut contenir un ou plusieurs cycles aromatiques, le radical R étant choisi parmi un groupement alkyle, allyle, aryle, linéaires ou ramifiés et R' représente un atome d'hydrogène ou un cation,
a représente le nombre de monomères, tel que la masse desdits polymères de formule (I) est supérieure à 2000 daltons,
x représente le taux de substitution des monomères A par des groupements X, x étant compris entre 20 et 150%,
y représente le taux de substitution des monomères A par des groupements Y, y étant compris entre 30 et 150%.

2. Composition pour son application selon la revendication 1, dans laquelle que ledit polymère biocompatible comprend des groupements chimiques fonctionnels Z, différents de X et Y, capables de conférer audit polymère des propriétés biologiques ou physicochimiques supplémentaires.

3. Composition pour son application selon la revendication 2, dans laquelle le taux de substitution de l'ensemble des monomères A par des groupements Z représenté par « z » est compris entre 0 et 50%.

4. Composition pour son application selon la revendication 2 ou 3, dans laquelle les groupements Z sont identiques ou différents et sont des acides aminés, des acides gras, des alcools gras, des céramides, ou des dérivés de ceux-ci, ou encore des séquences nucléotidiques d'adressage.

5. Composition pour son application selon la revendication 2 ou 3, dans laquelle les groupements Z sont identiques ou différents et sont des agents thérapeutiques.

6. Composition pour son application selon l'une quelconque des revendications précédentes, dans laquelle la composition pharmaceutique ou dermatologique sont destinés à prévenir, à soulager et/ou à traiter des démangeaisons induites par des lésions ou irritations chez un individu au niveau d'une zone de contact avec un milieu extérieur.

7. Composition pour son application selon la revendication 6, dans laquelle lesdites lésions et irritations sont choisies parmi les lésions cutanées, les lésions de la cornée, les lésions du tympan, les lésions du tractus digestif, les lésions du tractus respiratoire telles que les lésions des tissus des voies aériennes et pulmonaires et les lésions du tractus uro-génital.

8. Composition pour son application selon l'une quelconque des revendications 1 à 7, dans laquelle la composition pharmaceutique ou dermatologique est destiné prévenir, à soulager et/ou à traiter le prurit induit par
des brûlures cutanées profondes, particulièrement des brûlures profondes du deuxième degré ;
les cicatrices et le tissu cicatriciel ;
des ulcères de la peau et/ou des muqueuses et/ou de la cornée ;
des neuropathies périphériques et/ou dégénératives;
des boutons de fièvre ;
des gerçures, particulièrement des gerçures des doigts;
une hyperkératinisation de la peau, un psoriasis, un eczéma ou un zona ;
une opération chirurgicale
une radiothérapie ;
une lésion du tympan;
l'asthme et/ou les rhinites et/ou les obstructions des bronches ;
les aphtes et/ou les maux de gorge et/ou les douleurs dentaires ;
les arthroses ou les arthrites ;
les irritations des muqueuses et/ou de la peau;
les maladies chroniques **caractérisées par** une destruction et/ou un remodelage permanent de la matrice extracellulaire, comme par exemple les neuropathies périphériques et/ou dégénératives, le psoriasis, l'eczéma, le zona, l'asthme, les bronchites, les rhinites, les arthroses, les arthrites, la maladie de Crohn.

## Patentansprüche

1. Pharmazeutische, dermatologische oder kosmetische Zusammensetzung zur Anwendung als Medikament zur Prävention, Linderung und/oder Behandlung von Juckreiz und/oder zum Schutz von Geweben dagegen, wobei die Zusammensetzung ein biokompatibles Polymer der folgenden allgemeinen Formel (I) umfasst:
AaXxYy (I),
worin:
A für ein Monomer, das aus Glucose ausgewählt ist, steht,
X für eine RCOOR'-Gruppe steht,
Y für eine O- oder N-Sulfonatgruppe steht, die an A gebunden ist und einer der folgenden Formeln entspricht: -ROSO3R', -RNSO3R', worin:
R für eine gegebenenfalls verzweigte und/oder ungesättigte aliphatische Kohlenwasserstoffkette, die einen oder mehrere aromatische Ringe enthalten kann, steht, wobei der Rest R aus einer linearen oder verzweigten Alkyl-, Allyl- und Arylgruppe ausgewählt ist und R' für ein Wasserstoffatom oder ein Kation steht,
a für die Zahl von Monomeren steht und so beschaffen ist, dass die Masse der Polymere der Formel (I) über 2000 Dalton liegt,
x für den Substitutionsgrad der Monomere A durch Gruppen X steht, wobei x zwischen 20 und 150% liegt,
y für den Substitutionsgrad der Monomere A durch Gruppen Y steht, wobei y zwischen 30 und 150% liegt.

2. Zusammensetzung zur Anwendung nach Anspruch 1, wobei das biokompatible Polymer funktionelle chemische Gruppen Z umfasst, die von X und Y verschieden sind und dazu in der Lage sind, dem Polymer zusätzliche biologische oder physikalischchemische Eigenschaften zu verleihen.

3. Zusammensetzung zur Anwendung nach Anspruch 2, wobei der Substitutionsgrad aller Monomere A durch Gruppen Z durch "z" wiedergegeben wird und zwischen 0 und 50% liegt.

4. Zusammensetzung zur Anwendung nach Anspruch 2 oder 3, wobei die Gruppen Z gleich oder verschieden sind und Aminosäuren, Fettsäuren, Fettalkohole, Ceramide oder Derivate davon oder auch Zielsteuerungs-Nukleotidsequenzen sind.

5. Zusammensetzung zur Anwendung nach einem der Ansprüche 2 oder 3, wobei die Gruppen Z gleich oder verschieden sind und Therapeutika sind.

6. Zusammensetzung zur Anwendung nach einem der vorhergehenden Ansprüche, wobei die pharmazeutische oder dermatologische Zusammensetzung zur Prävention, Linderung und/oder Behandlung von durch Läsionen oder Reizungen induziertes Jucken bei einem Individuum in einem mit einem Außenmilieu in Kontakt stehenden Bereich vorgesehen ist.

7. Zusammensetzung zur Anwendung nach Anspruch 6, wobei die Läsionen und Reizungen aus Hautläsionen, Hornhautläsionen, Trommelfellläsionen, Läsionen des Verdauungstrakts, Läsionen des Respirationstrakts wie Läsionen von Luftwegs- und Lungengeweben und Läsionen des Urogenitaltrakts ausgewählt sind.

8. Zusammensetzung zur Anwendung nach einem der Ansprüche 1 bis 7, wobei die pharmazeutische oder dermatologische Zusammensetzung zur Prävention, Linderung und/oder Behandlung von durch tiefe Verbrennungen, insbesondere tiefe Verbrennungen zweiten Grades, Narben und Narbengewebe;
Geschwüren der Haut und/oder der Schleimhäute und/oder der Hornhaut;
peripheren und/oder degenerativen Neuropathien; Fieberbläschen;
Risse, insbesondere Risse der Finger; Hyperkeratinisierung der Haut, Psoriasis, Ekzem oder Zona;
einen chirurgischen Eingriff Strahlentherapie;
eine Trommelfellläsion;
Asthma und/oder Rhinitis und/oder Bronchienobstruktionen;
Aphthen und/oder Halsschmerzen und/oder Zahnschmerzen;
Arthrose oder Arthritis;
Reizungen der Schleimhäute und/oder der Haut;
chronische Erkrankungen, die durch eine Zerstörung und/oder permanente Remodellierung der extrazellulären Matrix gekennzeichnet sind, wie beispielsweise periphere und/oder degenerative Neuropathien, Psoriasis, Ekzem, Zona, Asthma, Bronchitis, Rhinitis, Arthrose, Arthritis, Morbus Crohn;
induzierten Juckreiz vorgesehen ist.

## Claims

1. Pharmaceutical, dermatological or cosmetic composition for its use as a medicament intended for the prevention, relief and/or treatment of pruritus and/or protection of the tissues against the latter, said composition of a biocompatible polymer corresponding to the following general formula (I)
AaXxYy (I)
in which:
A represents a monomer selected from glucose,
X represents a RCOOR' group,
Y represents an O or N-sulphonate group bound to A and corresponding to one of the following formulas - ROSO3R', -RNSO3R' in which:
R represents an aliphatic hydrocarbon chain, possibly branched and/or unsaturated and which may contain one or more aromatic rings and R' represents one hydrogen atom or one cation,
a represents the number of monomers, and is such that the mass of said polymers of formula (I) is greater than 2000 daltons.
x represents the rate of substitution of the A monomers by the X groups, x is between 20 and 150%,
y represents the rate of substitution of the A monomers by Y groups, y is between 30 and 150%,

2. Composition for its use according to claim 1, wherein said biocompatible polymer comprises functional chemical groups Z, different from X and Y and capable of bestowing additional biological or physical and chemical properties on the said polymers.

3. Composition for its use according to claim 3, wherein the rate of substitution of all the A monomers by Z groups represented by "z" is between 0 and 50%.

4. Composition for its use according to any one of claims 2 or 3, wherein the Z groups are identical or different and are amino acids, fatty acids, fatty alcohols, ceramides or derivatives of the latter, or furthermore addressing nucleotide sequences.

5. Composition for its use according to any one of claims 2 or 3, wherein the Z groups are identical or different and are therapeutic agents.

6. Composition for its use according to any of the preceding claims, wherein the pharmaceutical or dermatological composition is intended to prevent, relieve and/or treat itching induced by lesions or irritations in an individual in an area in contact with an outside medium.

7. Composition for its use according to claim 6, wherein said lesions and irritations are selected among skin lesions, corneal lesions, lesions of the eardrum, lesions of the digestive tract, lesions of the respiratory tract such as lesions of the tissues of the airways and lungs and lesions of the urogenital tract.

8. Composition for its use according to any of claim 1 to 7, wherein the pharmaceutical or dermatological composition is intended to prevent, relieve and/or treat pruritus induced by
deep skin burns, particularly deep second degree burns;
scars and cicatricial tissue;
ulcers of the skin and/or the mucosae and/or the cornea;
peripheral and/or degenerative neuropathies;
cold sores;
chapping, particularly chapping of the fingers;
hyperkeratinisation of the skin, psoriasis, eczema or herpes zoster;
a surgical operation;
radiotherapy;
a lesion of the eardrum;
asthma and/or rhinitis and/or bronchial obstruction;
aphthous ulcers and/or sore throats and/or dental pains; arthroses or arthritis;
irritation of the mucosae and/or the skin;
chronic diseases **characterised by** destruction and/or permanent remodelling of the extracellular matrix, such as for example peripheral and/or degenerative neuropathies, psoriasis, eczema, herpes zoster, asthma, bronchitis, rhinitis, arthroses, arthritis and Crohn's disease.
